# EUROPEAN PATENT APPLICATION

(11) **EP 3 907 215 A1**
(43) Date of publication of application: **10.11.2021**
(21) Application number: 19908010.2
(22) Date of filing: 02.01.2019
(51) Int. Cl.: C07C 67/30, C07C 69/76

(54) **SYNTHESIS METHOD FOR CANDESARTAN CILEXETIL INTERMEDIATE**

(71) Applicant: Linhai Huanan Chemical Co., Ltd., Zhejiang 317016 (CN); Zhejiang Huahai Pharmaceutical Co., Ltd., Taizhou, Zhejiang 317024 (CN)
(72) Inventor: TU, Guoliang, Zhejiang 317016 (CN); HUANG, Jiansheng, Zhejiang 317016 (CN); ZHANG, Lu, Zhejiang 317016 (CN); YANG, Tao, Zhejiang 317016 (CN); LIANG, Zunjun, Zhejiang 317016 (CN)
(74) Representative: Icosa
(86) International application number: PCT/CN2019/070055
(87) International publication number: WO 2020/140193

(57) **Abstract**

A synthesis method for a candesartan cilexetil intermediate represented by formula (II). The method comprises: (1) dissolving a compound represented by formula (IV) to an aprotic solvent to obtain a first mixed solution, and dissolving a phase transfer catalyst and an azidation reagent to water to obtain a second mixed solution; (2) dropping the first mixed solution to the second mixed solution for azidation reaction, and after the reaction is ended, standing and layering same to obtain an organic phase containing a compound represented by formula (V); (3) dropping the obtained organic phase containing the compound represented by formula (V) to tertiary butyl alcohol for rearrangement reaction, and after the reaction is ended, concentrating same to obtain a solid or oily material, then adding a crystallizing solvent to the obtained solid or oily material for recrystallization, and separating same to obtain a crystal, i.e., the candesartan cilexetil intermediate represented by formula (II). The synthesis method of the present application does not require the use of a DMF solvent, is safe and environmentally friendly, and reduces generation of ammonia nitrogen wastewater.

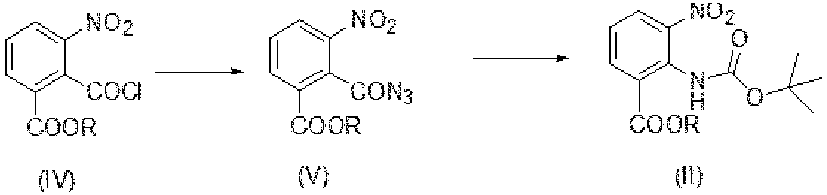

## Description

### FIELD OF THE INVENTION

The present application relates to a method for synthesizing candesartan cilexetil intermediate.

### BACKGROUND OF THE INVENTION

Candesartan cilexetil is an effective antihypertensive drug of angiotensin II receptor blockers with high selectivity. Since it increases the specificity and selectivity of angiotensin II receptor level blockade in the circulatory system and tissues, it has the advantages over angiotensin converting enzyme (ACE) inhibitors, with good antihypertensive effect and few adverse reactions. Candesartan cilexetil was jointly developed by Takeda Pharmaceutical Company in Japan and Atlas Copco in Sweden and was first launched in Sweden in November 1997, whose chemical name is (±)-2-ethoxy-1-[[2'-(1*H*-tetrazol-5-yl)[1,1'-biphenyl]-4-yl]methyl]-1*H*-benzimidazole-7-formic acid-1-[[(cyclohexyloxy)carbonyl]oxy]ethyl ester. Its chemical structural formula is represented by formula (I): 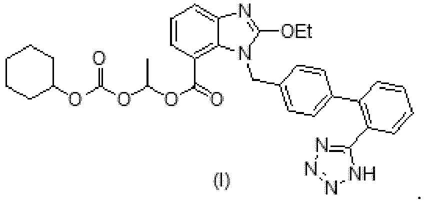 2-((tert-butoxycarbonyl)amino)-3-nitrobenzoate is a key intermediate for synthesizing candesartan cilexetil, whose structural formula is represented by formula (II): 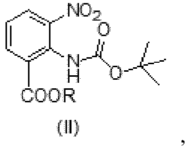 wherein R is methyl or ethyl group.

Literatures US5196444, WO2011145100 and WO2013186792 reported that 2-((tert-butoxycarbonyl)amino)-3-nitrobenzoate could be obtained by using 3-nitrophthalic acid as a raw material and carrying out esterification, acyl chlorination, azidation and rearrangement reactions. The synthesis route is as follows:

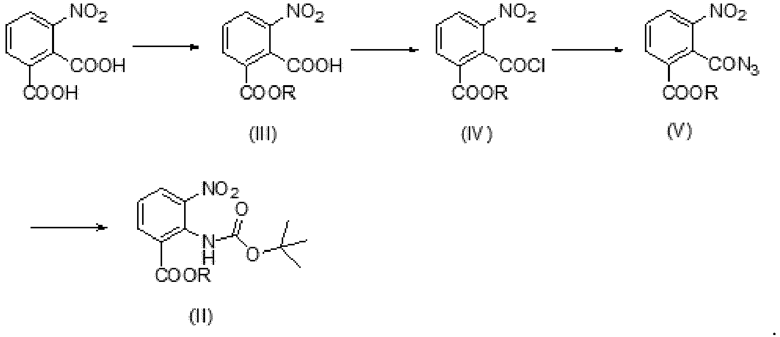

In these literatures, the acyl chloride compound of formula (IV) is reacted with sodium azide in the presence of DMF, washed, and distilled to obtain the acyl azide of formula (V) in solid form, which is reacted with tertiary butyl alcohol for rearrangement reaction to obtain 2-((tert-butoxycarbonyl)amino)-3-nitrobenzoate. In this synthesis process, DMF is used for azidation reaction, leading to generation of a great amount of ammonia nitrogen wastewater after washing, which is difficult to deal with and is not environmentally friendly. In addition, the acyl azide reaction solution reported in US5196444 is washed followed by extraction with a solvent and distillation. A major safety risk exists since acyl azide is thermal instability and the distillation will lead readily to material decomposition, which is not suitable for commercial production.

### SUMMARY OF THE INVENTION

The present application modifies the synthesis route in these literatures and provides a green, environmentally friendly, safe, efficient, economical and practical synthesis method of candesartan cilexetil intermediate.

The specific technical solution is as follows:
1. A method for synthesizing candesartan cilexetil intermediate represented by formula (II), 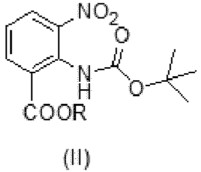 comprises:
   (1) dissolving a compound represented by formula (IV) in an aprotic solvent to obtain a first mixed solution; and dissolving a phase transfer catalyst and an azidation reagent in water to obtain a second mixed solution; preferably the azidation reagent is sodium azide or potassium azide;
   (2) adding the first mixed solution dropwise to the second mixed solution to perform an azidation reaction; after the azidation reaction is completed, standing and layering to obtain an organic phase containing a compound represented by formula (V); and
   (3) adding the organic phase containing the compound represented by formula (V) dropwise to tertiary butyl alcohol to perform a rearrangement reaction; after the rearrangement reaction is completed, concentrating the reaction solution to obtain a solid or an oily material, then adding a crystallizing solvent to the solid or the oily material for recrystallization, and separating to obtain a crystal, i.e., the candesartan cilexetil intermediate represented by formula (II); 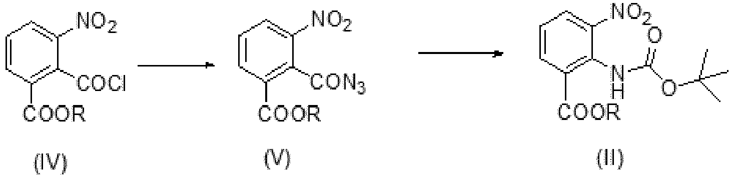 wherein, R is methyl or ethyl.

In some embodiments of the present application, the aprotic solvent is selected from the group consisting of toluene, chlorobenzene, xylene, chloroform, 1,2-dichloroethane and 1,2-dibromoethane, or any combination thereof, preferably toluene or chlorobenzene.

In some embodiments of the present application, the phase transfer catalyst is selected from the group consisting of tetrabutylammonium bromide, tetrabutylammonium chloride, tetrabutylammonium hydrogen sulfate, tetrabutylammonium fluoride and tetrabutylammonium iodide, or any combination thereof, preferably tetrabutylammonium bromide.

In some embodiments of the present application, the organic phase containing the compound represented by formula (V) in step (2) can be used directly in the next reaction without purification.

In some embodiments of the present application, in step (3), the solid or the oily material is obtained by distillation after the rearrangement reaction. Specifically, distillation can be atmospheric distillation or reduced pressure distillation, etc.

In some embodiments of the present application, a molar ratio of the phase transfer catalyst to the compound represented by formula (IV) is 0.01-0.08:1.

In some embodiments of the present application, the azidation reaction is carried out at -3°C to 10°C.

In some embodiments of the present application, the rearrangement reaction is carried out at 75°C to 95°C.

In some embodiments of the present application, a molar ratio of tertiary butyl alcohol to compound represented by formula (IV) is 1.0-5.0:1.

In some embodiments of the present application, the crystallizing solvent is selected from the group consisting of ethanol, methanol, isopropanol and ethyl acetate, or any combination thereof,
or
the crystallizing solvent is a mixed solution of at least one of ethanol, methanol, isopropanol or ethyl acetate with water.

In some embodiments of the present application, the compound represented by formula (IV) can be synthesized by the following method:
(a) subjecting 3-nitrophthalic acid to an esterification reaction to obtain a compound represented by formula (III); and
(b) subjecting the compound represented by formula (III) to an acyl chlorination reaction to obtain a compound represented by formula (IV); 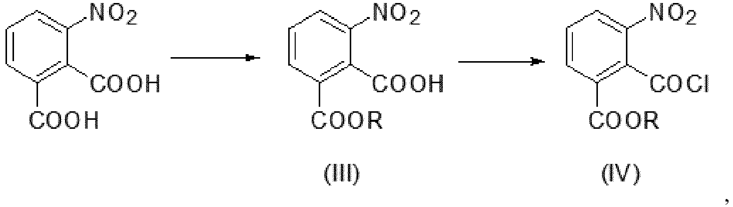 wherein, R is methyl or ethyl.

In some embodiments of the present application, in step (a), the esterification reaction is carried out by using methanol or ethanol; and in step (b), the acyl chlorination reaction is carried out by using thionyl chloride.

In some embodiments of the present application, the compound represented by formula (IV) can be synthesized by the method disclosed in the prior art, for example but not limited to a synthesis method of reference example 1 according to US5196444.

The present application provides a method for synthesizing candesartan cilexetil intermediate, having following advantages:
1. The azidation reaction is carried out in an aqueous solution and does not require the use of DMF solvent, which is safe and environmentally friendly and reduces the generation of ammonia nitrogen wastewater. After the reaction, the organic phase can be used directly in the next reaction, which is simple for operation;
2. The rearrangement reaction is carried out through adding dropwise, wherein the heat release and gases release can be controlled, which is safe to operate and suitable for large-scale production;
3. All organic solvents used can be recycled and can be produced with low costs and few wastes, which is environmentally friendly.

Certainly, it is not necessary to achieve all above advantages through carrying out any one of methods in the present application.

### DETAILED DESCRIPTION OF THE INVENTION

For the sake of clarity of the purpose, technical solutions and advantages of the present application, the application is further described in detail in combination with specific examples. Obviously, the specific examples are only a part of the examples of the present application, rather than all the examples. Based on the examples in the present application, all other examples obtained by those of ordinary skill in the art without creative work shall fall within the protection scope of the present application.

General test methods of the present application:
Instrument: high performance liquid chromatograph (HPLC) equipped with UV detector;
Chromatographic column: Agilent XDB C8 250^{∗}4.6mm 5µm or an equivalent chromatographic column;
Buffer: 1.36g potassium dihydrogen phosphate dissolved in 1000ml water, the pH was adjusted to 2.5 with concentrated phosphoric acid;
Mobile phase A: buffer: acetonitrile = 60: 40 (v/v);
Mobile phase B: buffer: acetonitrile = 15: 85 (v/v);
Flow rate: 1.0ml/min;
The temperature of the chromatographic column: 30°C;
Sample size: 10µl;
Detection wavelength: 210nm;
Gradient table:

| Time (min) | Mobile phase A (%v/v) | Mobile phase B (%v/v) |
|---|---|---|
| 0→15 | 100→0 | 0→100 |
| 15→27 | 0 | 100 |
| 27→27.5 | 0→100 | 100→0 |
| 27.5→35 | 100 | 0 |

### Preparation example of ethyl 2-chlorocarbonyl-3-nitrobenzoate

35g of 3-nitrophthalic acid, 300mL ethanol and 20mL concentrated sulfuric acid were added to a 500mL four-necked flask, heated to reflux, reacted for 24 hours and concentrated to remove ethanol, then 300mL saturated potassium carbonate aqueous solution was added, extracted with 50mL of dichloromethane, and an organic layer was discarded; the pH of an water layer was adjusted to 1 with hydrochloric acid, and the water layer was extracted with 2×300mL methylene chloride. The methylene chloride layers obtained by extracting the water layer twice were combined and concentrated to dryness to obtain 30.2g of ethyl 3-nitro-2-carboxybenzoate with a yield of 76.2%.

30.2g of ethyl 3-nitro-2-carboxybenzoate, 19.5g of thionyl chloride and 150mL toluene were added to a 500mL four-necked flask, heated to reflux, reacted for 2 hours and concentrated to dryness to obtain 32.2g of ethyl 2-chlorocarbonyl-3-nitrobenzoate with a yield of 99%.

### Preparation example of methyl 2-chlorocarbonyl-3-nitrobenzoate

According to the method described in the preparation example of ethyl 2-chlorocarbonyl-3-nitrobenzoate, the ethanol used in the esterification reaction was replaced by methanol to prepare methyl 2-chlorocarbonyl-3-nitrobenzoate.

### Example 1 Preparation of ethyl 2-((tert-butoxycarbonyl) amino) -3-nitrobenzoate

60mL water, 6.5g sodium azide (0.1mol) and 1.6g of tetrabutylammonium bromide (0.005mol) were added to a 250mL four-necked flask, cooled to 0°C and a toluene solution formed by dissolving 25.7g ethyl 2-chlorocarbonyl-3-nitrobenzoate in 140mL toluene was added dropwise. After the dropwise addition was completed, the reaction was maintained at 0°C for 1h, left stand and layered to obtain a toluene solution containing ethyl 2-(azidocarbonyl)-3-nitrobenzoate.

11.1g of tertiary butyl alcohol (0.15mol) was added to a 250mL four-necked flask and heated to 80°C. Then the toluene solution containing ethyl 2-(azidocarbonyl)-3-nitrobenzoate obtained above was added dropwise. During the addition process, a reaction temperature was controlled at 80 to 90°C. The reaction was maintained for 1h after the addition was completed. After an oily material was obtained by concentration under reduced pressure, 80mL ethanol was added and the temperature was reduced to 0°C. The mixture was stirred for 2 h, filtered and dried to obtain 27.9g of ethyl 2-((tert-butoxycarbonyl)amino)-3-nitrobenzoate with a yield of 90% and a purity of 97.2%.

### Example 2 Preparation of methyl 2-((tert-butoxycarbonyl) amino) -3-nitrobenzoate

60mL water, 6.5g sodium azide (0.1mol) and 1.6g of tetrabutylammonium bromide (0.005mol) were added to a 250mL four-necked flask, cooled to 0°C and a chlorobenzene solution formed by dissolving 24.2g methyl 2-chlorocarbonyl-3-nitrobenzoate in 140mL chlorobenzene was added dropwise. After the dropwise addition was completed, the reaction was maintained at 0°C for 1h, left stand and layered to obtain a chlorobenzene solution containing methyl 2-(azidocarbonyl)-3-nitrobenzoate.

11.1g of tertiary butyl alcohol (0.15mol) was added to a 250mL four-necked flask and heated to 80°C. Then the chlorobenzene solution containing methyl 2-(azidocarbonyl)-3-nitrobenzoate obtained above was added dropwise. During the addition process, a reaction temperature was controlled at 80 to 90°C. The reaction was maintained for 1h after the addition was completed. After a solid was obtained by concentration under reduced pressure, 80mL ethanol was added and the temperature was reduced to 0°C. The mixture was stirred for 2h, filtered and dried to obtain 27.5g of methyl 2-((tert-butoxycarbonyl)amino)-3-nitrobenzoate with a yield of 92.9% and a purity of 95.4%.

### Example 3 Preparation of methyl 2-((tert-butoxycarbonyl) amino) -3-nitrobenzoate

60mL water, 6.5g sodium azide (0.1mol) and 1.6g of tetrabutylammonium bromide (0.005mol) were added to a 250mL four-necked flask, cooled to 0°C and a 1,2-dichloroethane solution formed by dissolving 24.2g methyl 2-chlorocarbonyl-3-nitrobenzoate in 140mL 1,2-dichloroethane was added dropwise. After the dropwise addition was completed, the reaction was maintained at 0°C for 1h, left stand and layered to obtain a 1,2-dichloroethane solution containing methyl 2- (azidocarbonyl) -3- nitrobenzoate.

11.1g of tertiary butyl alcohol (0.15mol) was added to a 250mL four-necked flask and heated to 80°C. Then the 1,2-dichloroethane solution containing methyl 2-(azidocarbonyl)-3-nitrobenzoate obtained above was added dropwise. During the addition process, a reaction temperature was controlled at 80 to 90°C. The reaction was maintained for 1h after the addition was completed. After a solid was obtained by concentration under reduced pressure, 80mL ethanol was added and the temperature was reduced to 0°C. The mixture was stirred for 2h, filtered and dried to obtain 27.1g of methyl 2-((tert-butoxycarbonyl)amino)-3-nitrobenzoate with a yield of 91.5% and a purity of 96.5%.

### Example 4 Ethyl 2-((tert-butoxycarbonyl)amino)-3-nitrobenzoate

60mL water, 6.5g sodium azide (0.1mol) and 1.6g of tetrabutylammonium bromide (0.005mol) were added to a 250mL four-necked flask, cooled to -3°C and a toluene solution formed by dissolving 25.7g ethyl 2-chlorocarbonyl-3-nitrobenzoate in 140mL toluene was added dropwise. After the dropwise addition was completed, the reaction was maintained at -3°C for 2h, left stand and layered to obtain a toluene solution containing ethyl 2-(azidocarbonyl)-3-nitrobenzoate.

11.1g of tertiary butyl alcohol (0.15mol) was added to a 250mL four-necked flask and heated to 80°C. Then the toluene solution containing ethyl 2-(azidocarbonyl)-3-nitrobenzoate obtained above was added dropwise. During the addition process, a reaction temperature was controlled at 80 to 90°C. The reaction was maintained for 1h after the addition was completed. After an oily material was obtained by concentration under reduced pressure, 80mL ethanol was added and the temperature was reduced to 0°C. The mixture was stirred for 2 h, filtered and dried to obtain 27.1 g of ethyl 2-((tert-butoxycarbonyl)amino)-3-nitrobenzoate with a yield of 87.4% and a purity of 98.0%.

### Example 5 Ethyl 2-((tert-butoxycarbonyl)amino)-3-nitrobenzoate

60mL water, 6.5g sodium azide (0.1mol) and 1.6g of tetrabutylammonium bromide (0.005mol) were added to a 250mL four-necked flask, cooled to 10°C and a toluene solution formed by dissolving 25.7g ethyl 2-chlorocarbonyl-3-nitrobenzoate in 140mL toluene was added dropwise. After the dropwise addition was completed, the reaction was maintained at 10°C for 1h, left stand and layered to obtain a toluene solution containing ethyl 2-(azidocarbonyl)-3-nitrobenzoate.

11.1g of tertiary butyl alcohol (0.15mol) was added to a 250mL four-necked flask and heated to 80°C. Then the toluene solution containing ethyl 2-(azidocarbonyl)-3-nitrobenzoate obtained above was added dropwise. During the addition process, a reaction temperature was controlled at 80 to 90°C. The reaction was maintained for 1h after the addition was completed. After an oily material was obtained by concentration under reduced pressure, 80mL ethanol was added and the temperature was reduced to 0°C. The mixture was stirred for 2 h, filtered and dried to obtain 26.8 g of ethyl 2-((tert-butoxycarbonyl)amino)-3-nitrobenzoate with a yield of 86.5% and a purity of 96.8%.

### Example 6 Ethyl 2-((tert-butoxycarbonyl)amino)-3-nitrobenzoate

60mL water, 6.5g sodium azide (0.1mol) and 0.32g of tetrabutylammonium bromide (0.001mol) were added to a 250mL four-necked flask, cooled to 0°C and a toluene solution formed by dissolving 25.7g ethyl 2-chlorocarbonyl-3-nitrobenzoate in 140mL toluene was added dropwise. After the dropwise addition was completed, the reaction was maintained at 0°C for 2h, left stand and layered to obtain a toluene solution containing ethyl 2-(azidocarbonyl)-3-nitrobenzoate.

11.1g of tertiary butyl alcohol (0.15mol) was added to a 250mL four-necked flask and heated to 80°C. Then the toluene solution containing ethyl 2-(azidocarbonyl)-3-nitrobenzoate obtained above was added dropwise. During the addition process, a reaction temperature was controlled at 80 to 90°C. The reaction was maintained for 1h after the addition was completed. After an oily material was obtained by concentration under reduced pressure, 80mL ethanol was added and the temperature was reduced to 0°C. The mixture was stirred for 2 h, filtered and dried to obtain 27.2 g of ethyl 2-((tert-butoxycarbonyl)amino)-3-nitrobenzoate with a yield of 87.8% and a purity of 97.8%.

### Example 7 Ethyl 2-((tert-butoxycarbonyl)amino)-3-nitrobenzoate

60mL water, 6.5g sodium azide (0.1mol) and 2.57g of tetrabutylammonium bromide (0.008mol) were added to a 250mL four-necked flask, cooled to 0°C and a toluene solution formed by dissolving 25.7g ethyl 2-chlorocarbonyl-3-nitrobenzoate in 140mL toluene was added dropwise. After the dropwise addition was completed, the reaction was maintained at 0°C for 1h, left stand and layered to obtain a toluene solution containing ethyl 2- (azidocarbonyl) -3- nitrobenzoate.

11.1g of tertiary butyl alcohol (0.15mol) was added to a 250mL four-necked flask and heated to 80°C. Then the toluene solution containing ethyl 2-(azidocarbonyl)-3-nitrobenzoate obtained above was added dropwise. During the addition process, a reaction temperature was controlled at 80 to 90°C. The reaction was maintained for 1h after the addition was completed. After an oily material was obtained by concentration under reduced pressure, 80mL ethanol was added and the temperature was reduced to 0°C. The mixture was stirred for 2 h, filtered and dried to obtain 28.2 g of ethyl 2-((tert-butoxycarbonyl)amino)-3-nitrobenzoate with a yield of 90.9% and a purity of 98.0%.

Examples mentioned above are only the preferred examples of the present application and are not intended to limit the present application. Any modification, equivalent replacement, improvement, etc. made within the spirit and principle of the present application shall be included within the protection scope of the present application.

## Claims

1. A method for synthesizing candesartan cilexetil intermediate represented by formula (II), 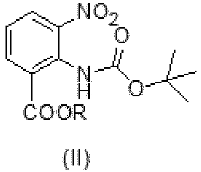 comprising:
(1) dissolving a compound represented by formula (IV) in an aprotic solvent to obtain a first mixed solution; and dissolving a phase transfer catalyst and an azidation reagent in water to obtain a second mixed solution; preferably the azidation reagent is sodium azide or potassium azide;
(2) adding the first mixed solution dropwise to the second mixed solution to perform an azidation reaction; after the azidation reaction is completed, standing and layering to obtain an organic phase containing a compound represented by formula (V); and
(3) adding the organic phase containing the compound represented by formula (V) dropwise to tertiary butyl alcohol to perform a rearrangement reaction; after the rearrangement reaction is completed, concentrating to obtain a solid or an oily material, then adding a crystallizing solvent to the solid or the oily material for recrystallization, and separating to obtain a crystal, i.e., the candesartan cilexetil intermediate represented by formula (II); 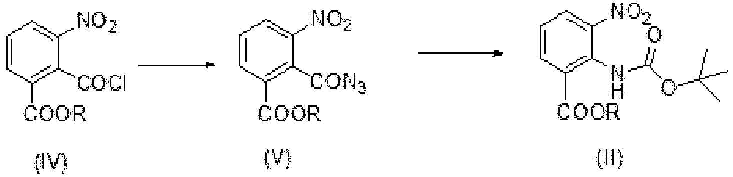 wherein, R is methyl or ethyl.

2. The method according to claim 1, wherein the aprotic solvent is selected from the group consisting of toluene, chlorobenzene, xylene, chloroform, 1,2-dichloroethane and 1,2-dibromoethane, or any combination thereof, preferably toluene or chloroform.

3. The method according to claim 1 or 2, wherein the phase transfer catalyst is selected from the group consisting of tetrabutylammonium bromide, tetrabutylammonium chloride, tetrabutylammonium hydrogen sulfate, tetrabutylammonium fluoride and tetrabutylammonium iodide, or any combination thereof, preferably tetrabutylammonium bromide.

4. The method according to any one of claims 1 to 3, wherein a molar ratio of the phase transfer catalyst to the compound represented by formula (IV) is 0.01-0.08:1.

5. The method according to any one of claims 1 to 4, wherein the azidation reaction is carried out at -3°C to 10°C.

6. The method according to any one of claims 1 to 5, wherein the rearrangement reaction is carried out at 75°C to 95°C.

7. The method according to any one of claims 1 to 6, wherein a molar ratio of tertiary butyl alcohol to the compound represented by formula (IV) is 1.0-5.0:1.

8. The method according to any one of claims 1 to 7, wherein the crystallizing solvent is selected from the group consisting of ethanol, methanol, isopropanol and ethyl acetate, or any combination thereof, or
the crystallizing solvent is a mixed solution of at least one of ethanol, methanol, isopropanol or ethyl acetate with water.

9. The method according to any one of claims 1 to 8, wherein the compound represented by formula (IV) is synthesized by the following method:
(a) subjecting 3-nitrophthalic acid to an esterification reaction to obtain a compound represented by formula (III); and
(b) subjecting the compound represented by formula (III) to an acyl chlorination reaction to obtain a compound represented by formula (IV); 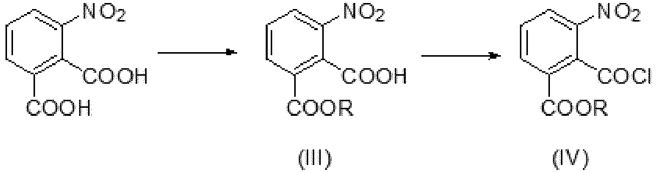 wherein, R is methyl or ethyl.

10. The method according to claim 9, wherein in step (a), the esterification reaction is carried out by using methanol or ethanol; and in step (b), the acyl chlorination reaction is carried out by using thionyl chloride.
